(19) Europäisches Patentamt / European Patent Office / Office européen des brevets

(11) **EP 2 971 177 B1**

(12) **EUROPEAN PATENT SPECIFICATION**

(45) Date of publication and mention of the grant of the patent:
**11.09.2019  Bulletin 2019/37**

(21) Application number: **14775449.3**

(22) Date of filing: **13.03.2014**

(51) Int Cl.:
*C12Q 1/6886* (2018.01)

(86) International application number:
**PCT/US2014/025860**

(87) International publication number:
**WO 2014/160120 (02.10.2014 Gazette 2014/40)**

(54) **COMPOSITIONS AND METHODS FOR DETECTING AND DETERMINING A PROGNOSIS FOR PROSTATE CANCER**

ZUSAMMENSETZUNGEN UND VERFAHREN ZUR ERKENNUNG UND BESTIMMUNG EINER PROGNOSE FÜR PROSTATAKREBS

COMPOSITIONS ET PROCÉDÉS POUR DÉTECTER ET DÉTERMINER UN PRONOSTIC DE CANCER DE LA PROSTATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **14.03.2013   US 201361785375 P**

(43) Date of publication of application:
**20.01.2016   Bulletin 2016/03**

(73) Proprietor: **Neogenomics Laboratories, Inc.**
**Fort Myers, FL 33913 (US)**

(72) Inventor: **ALBITAR, Maher**
**Irvine, CA 92618 (US)**

(74) Representative: **Bösl, Raphael Konrad**
**Patentanwälte**
**Isenbruck Bösl Hörschler PartG mbB**
**Prinzregentenstraße 68**
**81675 München (DE)**

(56) References cited:
EP-A2- 2 373 816      WO-A1-2010/065940
WO-A1-2012/115885      WO-A1-2012/135008
WO-A2-2005/113816      WO-A2-2006/056766

WO-A2-2012/092336      US-A1- 2008 286 814
US-A1- 2012 041 274      US-A1- 2013 058 925

• CELIA PRIOR ET AL: "Use of a combination of biomarkers in serum and urine to improve detection of prostate cancer", WORLD JOURNAL OF UROLOGY, SPRINGER, BERLIN, DE, vol. 28, no. 6, 15 July 2010 (2010-07-15), pages 681-686, XP019857852, ISSN: 1433-8726, DOI: 10.1007/S00345-010-0583-X

• WANLONG MA ET AL: "Diagnostic and Prognostic Scoring System for Prostate Cancer Using Urine and Plasma Biomarkers", GENETIC TESTING AND MOLECULAR BIOMARKERS, vol. 18, no. 3, 1 March 2014 (2014-03-01), pages 156-163, XP055207617, ISSN: 1945-0265, DOI: 10.1089/gtmb.2013.0424

• PRENSNER JOHN R ET AL: "Beyond PSA: The Next Generation of Prostate Cancer Biomarkers", SCIENCE TRANSLATIONAL MEDICINE, AMERICAN ASSOCIATION FOR THE ADVANCEMENT OF SCIENCE, WASHINGTON, DC, vol. 4, no. 127, 28 March 2012 (2012-03-28), pages 54-64, XP009168517, ISSN: 1946-6234, DOI: 10.1126/SCITRANSLMED.3003180

• ISABELLE GUYON ET AL: "A Four-Gene Expression Signature for Prostate Cancer Cells Consisting of UAP1, PDLIM5, IMPDH2, and HSPD1", UROTODAY INTERNATIONAL JOURNAL, vol. 02, no. 04, 1 August 2009 (2009-08-01), XP055036956, DOI: 10.3834/uij.1944-5784.2009.08.06

SEQUENCE LISTING

<110> ALBITAR, Maher

<120> COMPOSITIONS AND METHODS FOR DETECTING AND DETERMINING A PROGNOSIS FOR PROSTATE CANCER

<130> NGNL.P0002WO

<140> Unknown
<141> 2014-03-13

<150> 61/785,375
<151> 2013-03-14

<160> 15

<170> PatentIn version 3.5

<210> 1
<211> 23
<212> DNA
<213> Artificial sequence

<220>
<223> Synthetic primer

<400> 1
ttgcattcag aaaggagcag act          23

<210> 2
<211> 24
<212> DNA
<213> Artificial sequence

\<220\>
\<223\> Synthetic primer

\<400\> 2
caactggttc tgtagggttc gttt     24

\<210\> 3
\<211\> 18
\<212\> DNA
\<213\> Artificial sequence

\<220\>
\<223\> Synthetic primer

\<400\> 3
tggagcaaag gtggtaga     18

\<210\> 4
\<211\> 20
\<212\> DNA
\<213\> Artificial sequence

\<220\>
\<223\> Synthetic primer

\<400\> 4
aacctgtgac cacccctgaa     20

\<210\> 5
\<211\> 22
\<212\> DNA
\<213\> Artificial sequence

\<220\>
\<223\> Synthetic primer

\<400\> 5
tctttgtctc cgtttgcaga aa     22

\<210\> 6
\<211\> 17
\<212\> DNA
\<213\> Artificial sequence

\<220\>
\<223\> Synthetic primer

\<400\> 6
attgcacagg ttgctac     17

\<210\> 7
\<211\> 21
\<212\> DNA
\<213\> Artificial sequence

\<220\>
\<223\> Synthetic primer

<400> 7
ccacagtcat gatgggctct c     21

<210> 8
<211> 22
<212> DNA
<213> Artificial sequence

<220>
<223> Synthetic primer

<400> 8
ggatcccatc ggaaaagaag ta     22

<210> 9
<211> 15
<212> DNA
<213> Artificial sequence

<220>
<223> Synthetic primer

<400> 9
accactgagg cccct     15

<210> 10
<211> 21
<212> DNA
<213> Artificial sequence

<220>
<223> Synthetic primer

<400> 10
ccactgcatc aggaacaaaa g     21

<210> 11
<211> 22
<212> DNA
<213> Artificial sequence

<220>
<223> Synthetic primer

<400> 11
tgtgtcttca ggatgaaaca gg     22

<210> 12
<211> 16
<212> DNA
<213> Artificial sequence

<220>
<223> Synthetic primer

<400> 12
cgtgatcttg ctgggt     16

<210> 13
<211> 21
<212> DNA
<213> Artificial sequence

<220>
<223> Synthetic primer

<400> 13
ggaattcctg tgcatgaaag c          21

<210> 14
<211> 23
<212> DNA
<213> Artificial sequence

<220>
<223> Synthetic primer

<400> 14
cattcgaagt tcatcaaaga att          23

<210> 15
<211> 19
<212> DNA
<213> Artificial sequence

<220>
<223> Synthetic primer

<400> 15
cttcagcatt attccagtg          19


**Description**

This application claims the benefit of United States Provisional Patent Application No 61/785,375, filed March 14, 2013.

## INCORPORATION OF SEQUENCE LISTING

The sequence listing is part of the description.

## BACKGROUND OF THE INVENTION

### 1. Field of the Invention

The present invention relates generally to the field of cancer biology. More particularly, it concerns methods for detecting the presence of and determining the aggressiveness of prostate cancer.

### 2. Description of Related Art

Prostate cancer is the second most common cancer in men after lung cancer and its incidence is increasing due to the aging population. It is also the second leading cause of cancer-related death in men. The current screening methods for prostate cancer are based on measuring serum Prostate Specific Antigen (PSA). A PSA level ≥4.0 ng per milliliter has been the general threshold for a biopsy referral. Elevated PSA levels have been known to falsely indicate the possible presence of prostate cancer since it is also characteristic of Benign Prostatic Hyperplasia (BPH) due to the correlation between PSA level and prostate size. Relying on PSA levels leads to 75% false positive and too many unnecessary biopsies. More importantly, even when prostate cancer is detected, the clinical behavior of this cancer varies significantly and the disease can be lethal in some patients but indolent in others. Current data suggests that by relying on serum PSA, some patients are overtreated, therefore, it has been suggested that PSA testing may cause more harm due to the side effects that may result from unnecessary prostatectomy. Gleason histologic grading of prostate cancer remains the most reliable predictor of its clinical behavior. Convincing data demonstrates that similar outcome is obtained whether patients were treated or not when their tumor had Gleason Score 6.
Many attempts have been made to improve on serum PSA in its clinical utility. Free and complex PSA and isoforms of PSA have been used as an adjunct to PSA and they show some improvement in sensitivity and specificity, especially in cases in which patients are considered in the "grey zone," but all these remain inadequate in improving the prediction of cancer in patients with BPH. PSA velocity and doubling time are also used and showed some improvement, but this improvement remains limited. There is a need to improve on the PSA level screening not only in predicting the presence of cancer to avoid unnecessary biopsies, but also to develop a test that can also predict the clinical behavior of prostate cancer. Prior *et al.* (2010) disclose the use of a combination of biomarkers in serum and urine to improve detection of prostate cancer. Biomarkers analyzed were AMACR and MMP-2 levels, and GSTP1/RASSF1A methylation status, in addition to PSA levels. EP 2 373 816 concerns methods for screening, predicting and monitoring prostate cancer using four or more biomarkers selected from PDZ and LIM5, UAP1/AgX1 antigen, HSPD1/chaperonin, F5, IMPDH2, PPIB, RGS10 and PYCR1. WO 2012/115885 concerns circulating biomarkers. Prensner *et al.* (2012) is a review article about the next generation of prostate cancer biomarkers beyond PSA. Guyon *et al.* (2009) disclose a four-gene expression signature for prostate cancer cells consisting of UAP1, PDLIM5, IMPDH2, and HSPD1. WO 2010/065940 concerns materials and methods for determining diagnosis and prognosis of prostate cancer. WO 2006/056766 provides a method for determining the presence of prostate cancer by determining the level of expression of one or more markers in a blood sample from a subject comprising at least one of EM, c-met, pRB, EZH2, e-cad, CAXII, CAIX, HIF-1α, Jagged, PIM-1, hepsin, RECK, Clusterin, MMP9, MTSP-1, MMP24, MMP15, IGFBP-2, IGFBP-3, E2F4, caveolin, EF-1A, Kallikrein 2, Kallikrein 3 and PSGR. WO 2005/113816 concerns methods of diagnosing or treating prostate cancer using the ERG gene, alone or in combination with other over- or under-expressed genes in prostate cancer. WO 2012/092336 provides methods and systems of molecular profiling of diseases, such as cancer.

## SUMMARY OF THE INVENTION

Generally, the instant invention provides a set of blood and urine markers that can be used for highly accurate detection of prostate cancer and determination of prostate cancer aggressiveness.
Thus, in one embodiment, there is provided a method of detecting if a subject is at risk for prostate cancer or aggressive prostate cancer, comprising (a) obtaining a biological sample and urine sample from the subject; (b) measuring the mRNA expression levels of a set of the blood and urine markers consisting of the UAP1, PDLIM5, IMPDH2, HSPD1,

PCA3, PSA, TMPRSS2, ERG, GAPDH, B2M, PTEN and AR genes from the urine sample and from the blood sample; and (c) identifying the subject as at risk or not at risk for prostate cancer or aggressive prostate cancer based on the expression level of said genes.

In certain aspects of the embodiments, a subject has or is diagnosed with a prostate cancer. Thus, a method can comprise identifying a subject having a cancer as at risk or not at risk for an aggressive prostate cancer. In certain aspects, the subject has previously has a prostatectomy. In further aspects, the subject has or is diagnosed with an enlarged prostate or benign prostate hyperplasia (BPH).

In some aspect of the embodiments, identifying the subject as at risk or not at risk for prostate cancer or aggressive prostate cancer is based on the expression levels of the measured genes and the age of the subject. In one aspect, identifying the subject as at risk or not at risk for prostate cancer or aggressive prostate cancer further comprises correlating the expression levels of said genes with a risk for prostate cancer or aggressive prostate cancer. Such a correlating step can, in some case, be performed by a computer. In some aspects, an algorithm is used, that weights the relative predictive values of measured expression levels of the indicated genes. Examples of such algorithms are provided herein. In some cases, identifying the subject as at risk or not at risk for prostate cancer or aggressive prostate cancer further comprises analysis of the expression levels of said genes using a SVM, logistic regression, lasso, boosting, bagging, random forest, CART, or MATT algorithm. Such an analysis may, in some cases, be performed by a computer.

A sample for use according to the embodiments is a blood sample and urine sample. In this aspects, the method comprises obtaining (either directly or from a third party) a sample of blood and urine sample from the subject.

In a further aspect, the method further comprises in (b) measuring the expression levels of the genes in the sample and measuring the expression levels of the genes in a reference sample; and in (c) identifying the subject as at risk or not at risk for prostate cancer or aggressive prostate cancer by comparing the expression level of the genes in the sample from the subject to the expression level of the genes in the reference sample.

In some aspects, measuring the expression of said genes comprises measuring protein expression levels. Measuring protein expression levels may comprise, for example, performing an ELISA, Western blot or binding to an antibody array. In another aspect, measuring expression of said genes comprises measuring RNA expression levels. Measuring RNA expression levels may comprise performing RT-PCR, Northern blot or an array hybridization. Preferably, measuring the expression level of the genes comprises performing RT-PCR *(e.g.,* real time RT-PCR).

In some aspects, a method further comprises reporting whether the subject has a prostate cancer or has an aggressive prostate cancer. Reporting may comprise preparing an oral, written or electronic report. Thus, providing a report may comprise providing the report to the patient, a doctor, a hospital, or an insurance company.

The present disclosure also provides a method of treating a subject comprising selecting a subject identified as at risk for a prostate cancer or an aggressive prostate cancer in accordance with the method of the invention and administering an anti- cancer therapy the subject. In certain aspects, the anti-cancer therapy is a chemotherapy, a radiation therapy, a hormonal therapy, a targeted therapy, an immunotherapy or a surgical therapy *(e.g.,* prostatectomy).

In another embodiment, the present disclosure provides a method of selecting a subject for a diagnostic procedure comprising carrying out the method of the invention; and (c) performing a diagnostic procedure on the subject. For example, the diagnostic procedure can be a biopsy.

In still another embodiment, the present disclosure provides a method of determining a prognosis for a subject having a prostate cancer, comprising carrying out the method of the invention; and (c)

identifying the subject as having or not having an aggressive prostate cancer based on the expression level of said genes.

In yet a further embodiment, the present invention concerns a tangible computer-readable medium comprising computer-readable code that, when executed by a computer, causes the computer to perform operations comprising (a) receiving information corresponding to a level of expression of the UAP1, PDLIM5, IMPDH2, HSPD1, PCA3, PSA, TMPRSS2, ERG, GAPDH, B2M, PTEN and AR genes in a sample from a subject; and (b) determining a relative level of expression of said genes compared to a reference level, wherein altered expression of said genes compared to a reference level indicates that the subject is at risk of having prostate cancer or aggressive prostate cancer.

In one aspect, the tangible computer-readable medium further comprises receiving information corresponding to a reference level of expression of UAP1, PDLIM5, IMPDH2, HSPD1, PCA3, PSA, TMPRSS2, ERG, GAPDH, B2M, PTEN and AR in a sample from a healthy subject. In yet another aspect, the tangible computer-readable medium further comprises computer-readable code that, when executed by a computer, causes the computer to perform one or more additional operations comprising: sending information corresponding to the relative level of expression of said genes to a tangible data storage device. In yet another aspect, the computer-readable code is a code that, when executed by a computer, causes the computer to perform operations further comprising (c) calculating a diagnostic score for the sample, wherein the diagnostic score is indicative of the probability that the sample is from a subject having prostate cancer or aggressive prostate cancer. In one aspect, calculating a diagnostic score for the sample comprises using a SVM, logistic regression, lasso, boosting, bagging, random forest, CART, or MATT algorithm.

In still a further aspect, the reference level is stored in said tangible computer- readable medium. In another aspect, receiving information comprises receiving from a tangible data storage device information corresponding to a level of expression of said genes in a sample from a subject.

As used herein the specification, "a" or "an" may mean one or more. As used herein in the claim(s), when used in conjunction with the word "comprising", the words "a" or "an" may mean one or more than one.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more.

Throughout this application, the term "about" is used to indicate that a value includes the inherent variation of error for the device, the method being employed to determine the value, or the variation that exists among the study subjects.

Other objects, features and advantages of the present invention will become apparent from the following detailed description. It should be understood, however, that the detailed description and the specific examples, while indicating preferred embodiments of the invention, are given by way of illustration only.

## BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

**FIG. 1.** AUC (FIG. 1A) and error rate (FIG. 1B) using various algorithms in the training set. The contribution of each of the six variables included in the algorithms is also shown (FIG. IC).

**FIG. 2.** Using the test set of samples, the AUC (FIG. 2A) and error rate (FIG. 2B) are shown with various algorithms.

**FIG. 3.** Determining the cut-off point for distinguishing cancer patients from BPH. The middle dashed line is at 0.565 and the left and right dashed lines are at 0.55 and 0.58, respectively.

**FIG. 4.** AUC (FIG. 4A) and error rate (FIG. 4B) using various algorithms in the training set. The contribution of each of the four variables included in the algorithms is also shown (FIG. 4C).

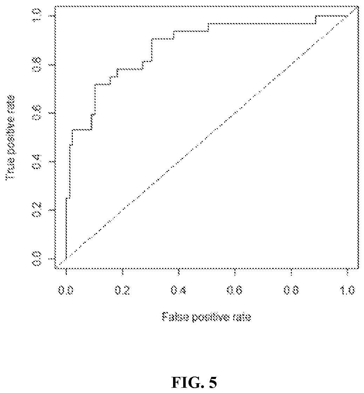

**FIG. 5.** ROC curve in distinguishing aggressive prostate cancer from BPH/Gleason <7.

**FIG. 6.** Combined scoring system utilizing both models (cancer vs. no cancer and aggressive cancer vs. BPH/indolent cancer) for prediction. Each square represents a patient. The distribution of the patients are shown in the top two rows. 75% with concordance results (Sensi = 68%, Spec = 99%). 25% Pog/Neg: mixed: neg/positive <7/positive $\geq$7.

**FIG. 7.** ROC curve of assay data for distinguishing PCa from BPH. Markers used in the analysis were (1) serum PSA protein level; (2) plasma ERG mRNA level; (3) plasma AR mRNA level; (4) urine PCA3 mRNA level; (5) urine PTEN level; (6) urine B2M mRNA level; (7) plasma B2M mRNA level; and (8) plasma GAPDH mRNA level

**FIG. 8.** ROC curves of assay data for distinguishing aggressive prostate cancer from BPH/Gleason <7. Curves show results when different numbers of markers were used (*i.e.,* Step 0 is 1 marker; Step 1 is two markers; Step 2 is three markers ect...). Markers used in the Step 8 curve, which achieved an AUROC of 0.79777, were (1) serum PSA protein level; (2) Age; (3) urine PSA; (4) plasma ERG mRNA level; (5) urine GAPDH mRNA level; (6) urine B2M mRNA level; (7) urine PTEN mRNA level; (8) urine PCA3 mRNA level; and (9) urine PDLIM5 mRNA level.

## DESCRIPTION OF ILLUSTRATIVE EMBODIMENTS

Disclosed here in are two algorithms, one for predicting the presence of prostate cancer in patients with benign prostate hyperplasia (BPH) and the second for predicting the presence of aggressive prostate cancer (Gleason $\geq$7). These algorithms were developed by assaying a combination of biomarkers isolated from both urine and plasma by real-time PCR, including UAP1, PDLIM5, IMPDH2, HSPD1, PCA3, PSA, TMPRSS2, ERG, GAPDH, and B2M. Therefore, the present disclosure provides a scoring system that takes advantage of two algorithms for detecting aggressive prostate cancer. This scoring system provides highly precise prediction (99% specificity and 68% sensitivity) of the presence of aggressive prostate cancer in 75% of patients. In 25% of patients, only the presence of cancer at 88% specificity and 67% sensitivity can be predicted, but not aggressiveness of the disease. This approach can be used to determine whether or not a patient needs a biopsy as well as when there is a doubt that the biopsy may be unrepresentative.

The first algorithm predicted cancer with an AUC of 0.77 in the training set and an AUC of 0.78 in test set. The overall specificity and sensitivity were 88% and 67%, respectively. The second algorithm predicted patients with a Gleason ≥7 with a significantly better AUC of 0.87 in the training set and an AUC of 0.88 in the test set (99% specificity and 47% sensitivity). By incorporating the two models in a scoring system, 75% of patients showed concordance between the two models. In concordant patients via both models, the prediction of the Gleason ≥7 was at a specificity of 99% and sensitivity of 68%. In patients showing discordance between the two models, predicting the aggressiveness of the disease was not accurate and only the first model predicting cancer vs. no cancer can be used.

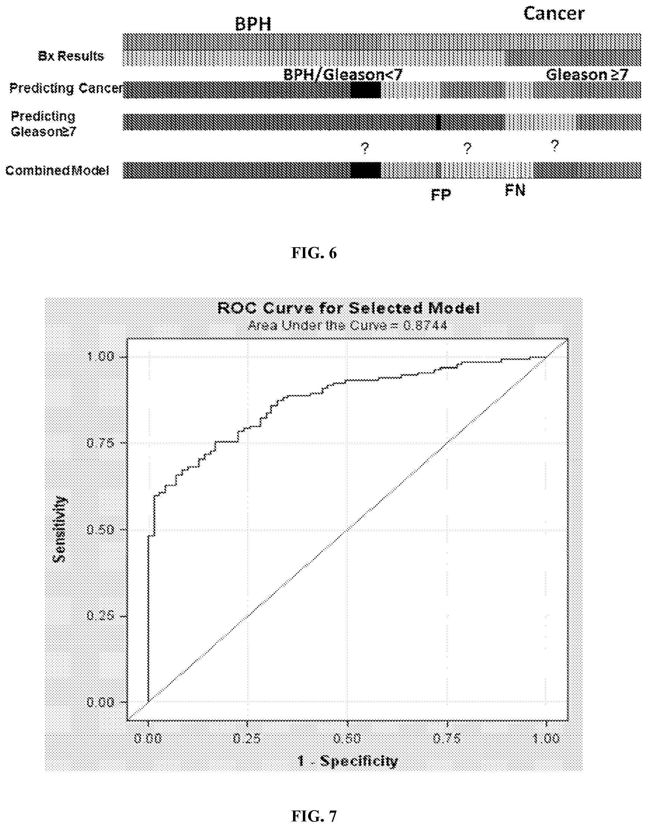

The assays were then further developed with the incorporation of two additional markers (AR and PTEN mRNA levels). Again assays were developed for (I) determining PCa vs. BPH; and (II) high-risk PCa (GS ≥7) vs. low-risk cancer (GS <7) or BPH. For the first of these analyses (to distinguishing PCa from BPH) the markers used were (1) serum PSA protein level; (2) plasma ERG mRNA level; (3) plasma AR mRNA level; (4) urine PCA3 mRNA level; (5) urine PTEN level; (6) urine B2M mRNA level; (7) plasma B2M mRNA level; and (8) plasma GAPDH mRNA level. Using these markers PCa could be distinguished from BPH with AUROC of 0.87. The testing set for this model showed sensitivity of 76% and specificity of 71% upon using a cut-off point of 0.64 (see, *e.g.,* FIG. 7 and Table 5). The second analysis (to distinguish high-risk PCa (GS ≥7) vs. GS <7 cancer or BPH) was developed using the markers: (1) serum PSA protein level; (2) Age; (3) urine PSA; (4) plasma ERG mRNA level; (5) urine GAPDH mRNA level; (6) urine B2M mRNA level; (7) urine PTEN mRNA level; (8) urine PCA3 mRNA level; (9) urine PDLIM5 mRNA level; and, optionally, (10) plasma PCA3 mRNA level; (11) plasma B2M mRNA level and (12) plasma HSPD1 mRNA level. With these markers high-risk PCa could be distinguished from low-grade cancer (GS <7) or BPH with an AUROC of 0.80.

Furthermore, by combining the results of the two analysis described *supra* a highly specific and sensitive diagnosis can be achieved (without the need to a biopsy). In the case where both analyses negative there is a high probability of no cancer and, in any case, a very low probability of high-risk cancer. Such subjects could therefore forego more invasive diagnostics, such as biopsy, and would require less frequent monitoring. On the other hand, when both analyses are positive there is a high probability that the subject has cancer and that the cancer is aggressive. These subjects would be subjected to biopsy and/or (aggressive) anti-cancer therapy, such as surgical resection. Likewise, if assays indicate that a subject is "PCa negative" but positive for high-risk cancer, the subject has a high probability of having cancer and that the cancer is high-risk. Again, these subjects would be subjected to biopsy and/or (aggressive) anti-cancer therapy. In the case of a patient indicated as "PCa positive," but negative for high-risk PCa, the patient has a high probability of having cancer, but the cancer is unlikely to be high-risk. These subjects could be subjected biopsy, but would not likely require immediate aggressive therapy or monitoring.

Thus, the newly developed assays and analyses are particularly helpful in determining the need to perform a prostate biopsy and may help in monitoring patients on active surveillance and in predicting progression. However, this prediction of the presence and aggressiveness of PCa is based on biopsy results.

In particular, the urine and plasma expression markers identified herein include:

- PDZ and LIM domain 5 (PDLIM5) see, *e.g.,* NCBI accession nos. NM_006457.4, NM_001011513.3, NM_001011515.2, NM_001011516.2, NM_001256425.1, NM_001256426.1, NM_001256427.1, NM 001256428.1, NR_046186.1 and NM_001256429.1, incorporated herein by reference.

- transmembrane protease, serine 2 (TMPRSS2) see *e.g.,* NCBI accession nos. NM_001135099.1 and NM_005656.3, incorporated herein by reference.

- UDP-N-acteylglucosamine pyrophosphorylase 1 (UAP1) see *e.g.,* NCBI accession no. NM_003115.4, incorporated herein by reference.

- IMP (inosine 5'-monophosphate) dehydrogenase 2 (IMPDH2) see *e.g.,* NCBI accession no. NM_000884.2, incorporated herein by reference.

- heat shock 60kDa protein 1 (chaperonin) (HSPD1) see *e.g.,* NCBI accession nos. NM_002156.4; and NM_199440.1, incorporated herein by reference.

- prostate cancer antigen 3 (PCA3) see *e.g.,* NCBI accession no. NR_015342.1, incorporated herein by reference.

- PSA or kallikrein-related peptidase 3 (KLK3) see *e.g.,* NCBI accession nos. NM_001030047.1, NM_001030048.1, and NM 001648.2, incorporated herein by reference.

- v-ets erythroblastosis virus E26 oncogene homolog (ERG) see *e.g.,* NCBI accession nos. NM_001136154.1, NM_001136155.1, NM_001243428.1, NM_001243429.1, NM_001243432.1, NM_004449.4, and NM_182918.3, incorporated herein by reference.

- PTEN or phosphatase and tensin homolog see *e.g.,* NCBI accession no. NM_000314.4, incorporated herein by reference.

- AR or androgen receptor, see *e.g.,* NCBI accession no. NM_000044.3, and NM_001011645.2 incorporated herein by reference.

- glyceraldehyde-3-phosphate dehydrogenase (GAPDH) see *e.g.,* NCBI accession nos. NM_001256799.1, and NM_002046.4, incorporated herein by reference.

- beta-2-microglobulin (B2M) see *e.g.,* NCBI accession no. NM_004048.2, incorporated herein by reference.

## I. Biomarker Detection

The expression of biomarkers or genes may be measured by a variety of techniques that are well known in the art. Quantifying the levels of the messenger RNA (mRNA) of a biomarker may be used to measure the expression of the biomarker. Alternatively, quantifying the levels of the protein product of a biomarker may be used to measure the expression of the biomarker. Additional information regarding the methods discussed below may be found in Ausubel *et al.* (2003) or Sambrook *et al.* (1989). One skilled in the art will know which parameters may be manipulated to optimize detection of the mRNA or protein of interest.

In some embodiments, said obtaining expression information may comprise RNA quantification, *e.g.,* cDNA microarray, quantitative RT-PCR, *in situ* hybridization, Northern blotting or nuclease protection. Said obtaining expression information may comprise protein quantification, *e.g.,* protein quantification comprises immunohistochemistry, an ELISA, a radioimmunoassay (RIA), an immunoradiometric assay, a fluoroimmunoassay, a chemiluminescent assay, a bioluminescent assay, a gel electrophoresis, a Western blot analysis, a mass spectrometry analysis, or a protein microarray.

A nucleic acid microarray may be used to quantify the differential expression of a plurality of biomarkers. Microarray analysis may be performed using commercially available equipment, following manufacturer's protocols, such as by using the Affymetrix GeneChip® technology (Santa Clara, CA) or the Microarray System from Incyte (Fremont, CA). For example, single-stranded nucleic acids (*e.g.*, cDNAs or oligonucleotides) may be plated, or arrayed, on a microchip substrate. The arrayed sequences are then hybridized with specific nucleic acid probes from the cells of interest. Fluorescently labeled cDNA probes may be generated through incorporation of fluorescently labeled deoxynucleotides by reverse transcription of RNA extracted from the cells of interest. Alternatively, the RNA may be amplified by *in vitro* transcription and labeled with a marker, such as biotin. The labeled probes are then hybridized to the immobilized nucleic acids on the microchip under highly stringent conditions. After stringent washing to remove the non-specifically bound probes, the chip is scanned by confocal laser microscopy or by another detection method, such as a CCD camera. The raw fluorescence intensity data in the hybridization files are generally preprocessed with the robust multichip average (RMA) algorithm to generate expression values.

Quantitative real-time PCR (qRT-PCR) may also be used to measure the differential expression of a plurality of biomarkers. In qRT-PCR, the RNA template is generally reverse transcribed into cDNA, which is then amplified via a PCR reaction. The amount of PCR product is followed cycle-by-cycle in real time, which allows for determination of the initial concentrations of mRNA. To measure the amount of PCR product, the reaction may be performed in the presence of a fluorescent dye, such as SYBR Green, which binds to double-stranded DNA. The reaction may also be performed with a fluorescent reporter probe that is specific for the DNA being amplified.

A non-limiting example of a fluorescent reporter probe is a TaqMan® probe (Applied Biosystems, Foster City, CA). The fluorescent reporter probe fluoresces when the quencher is removed during the PCR extension cycle. Multiplex qRT-PCR may be performed by using multiple gene-specific reporter probes, each of which contains a different fluorophore. Fluorescence values are recorded during each cycle and represent the amount of product amplified to that point in the amplification reaction. To minimize errors and reduce any sample-to-sample variation, qRT-PCR may be performed using a reference standard. The ideal reference standard is expressed at a constant level among different tissues, and is unaffected by the experimental treatment. Suitable reference standards include, but are not limited to, mRNAs for the housekeeping genes glyceraldehyde-3-phosphate-dehydrogenase (GAPDH) and $\beta$-actin. The level of mRNA in the original sample or the fold change in expression of each biomarker may be determined using calculations well known in the art.

Immunohistochemical staining may also be used to measure the differential expression of a plurality of biomarkers. This method enables the localization of a protein in the cells of a tissue section by interaction of the protein with a specific antibody. For this, the tissue may be fixed in formaldehyde or another suitable fixative, embedded in wax or plastic, and cut into thin sections (from about 0.1 mm to several mm thick) using a microtome. Alternatively, the tissue may be frozen and cut into thin sections using a cryostat. The sections of tissue may be arrayed onto and affixed to a solid surface (*i.e.,* a tissue microarray). The sections of tissue are incubated with a primary antibody against the antigen of interest, followed by washes to remove the unbound antibodies. The primary antibody may be coupled to a detection system, or the primary antibody may be detected with a secondary antibody that is coupled to a detection system. The detection system may be a fluorophore or it may be an enzyme, such as horseradish peroxidase or alkaline phosphatase, which can convert a substrate into a colorimetric, fluorescent, or chemiluminescent product. The stained tissue sections are generally scanned under a microscope. Because a sample of tissue from a subject with cancer may be heterogeneous, *i.e.,* some cells may be normal and other cells may be cancerous, the percentage of positively stained cells in the tissue may be determined. This measurement, along with a quantification of the intensity of staining, may be used to generate an expression value for the biomarker.

An enzyme-linked immunosorbent assay, or ELISA, may be used to measure the differential expression of a plurality of biomarkers. There are many variations of an ELISA assay. All are based on the immobilization of an antigen or antibody on a solid surface, generally a microtiter plate. The original ELISA method comprises preparing a sample containing the biomarker proteins of interest, coating the wells of a microtiter plate with the sample, incubating each well with a primary antibody that recognizes a specific antigen, washing away the unbound antibody, and then detecting the antibody-antigen complexes. The antibody-antibody complexes may be detected directly. For this, the primary antibodies are conjugated to a detection system, such as an enzyme that produces a detectable product. The antibody-antibody complexes may be detected indirectly. For this, the primary antibody is detected by a secondary antibody that is conjugated to a detection system, as described above. The microtiter plate is then scanned and the raw intensity data may be converted into expression values using means known in the art.

An antibody microarray may also be used to measure the differential expression of a plurality of biomarkers. For this, a plurality of antibodies is arrayed and covalently attached to the surface of the microarray or biochip. A protein extract containing the biomarker proteins of interest is generally labeled with a fluorescent dye or biotin. The labeled biomarker proteins are incubated with the antibody microarray. After washes to remove the unbound proteins, the microarray is scanned. The raw fluorescent intensity data may be converted into expression values using means known in the art.

Luminex multiplexing microspheres may also be used to measure the differential expression of a plurality of biomarkers. These microscopic polystyrene beads are internally color-coded with fluorescent dyes, such that each bead has a unique spectral signature (of which there are up to 100). Beads with the same signature are tagged with a specific oligonucleotide or specific antibody that will bind the target of interest (*i.e.,* biomarker mRNA or protein, respectively). The target, in turn, is also tagged with a fluorescent reporter. Hence, there are two sources of color, one from the bead and the other from the reporter molecule on the target. The beads are then incubated with the sample containing the targets, of which up to 100 may be detected in one well. The small size/surface area of the beads and the three dimensional exposure of the beads to the targets allows for nearly solution-phase kinetics during the binding reaction. The captured targets are detected by high-tech fluidics based upon flow cytometry in which lasers excite the internal dyes that identify each bead and also any reporter dye captured during the assay. The data from the acquisition files may be converted into expression values using means known in the art.

*In situ* hybridization may also be used to measure the differential expression of a plurality of biomarkers. This method permits the localization of mRNAs of interest in the cells of a tissue section. For this method, the tissue may be frozen, or fixed and embedded, and then cut into thin sections, which are arrayed and affixed on a solid surface. The tissue sections are incubated with a labeled antisense probe that will hybridize with an mRNA of interest. The hybridization and washing steps are generally performed under highly stringent conditions. The probe may be labeled with a fluorophore or a small tag (such as biotin or digoxigenin) that may be detected by another protein or antibody, such that the labeled hybrid may be detected and visualized under a microscope. Multiple mRNAs may be detected simultaneously, provided each antisense probe has a distinguishable label. The hybridized tissue array is generally scanned under a microscope. Because a sample of tissue from a subject with cancer may be heterogeneous, *i.e.,* some cells may be normal and other cells may be cancerous, the percentage of positively stained cells in the tissue may be determined. This measurement, along with a quantification of the intensity of staining, may be used to generate an expression value for each biomarker.

In a further embodiment, the marker level may be compared to the level of the marker from a control, wherein the control may comprise one or more tumor samples taken from one or more patients determined as having a certain metastatic tumor or not having a certain metastatic tumor, or both.

The control may comprise data obtained at the same time (*e.g.*, in the same hybridization experiment) as the patient's individual data, or may be a stored value or set of values, *e.g.,* stored on a computer, or on computer-readable media. If the latter is used, new patient data for the selected marker(s), obtained from initial or follow-up samples, can be compared to the stored data for the same marker(s) without the need for additional control experiments.

*Statistical analysis of marker expression*

As further detailed herein, once measurement of expression levels have been obtained for a sample the measurements can be applied to an algorithm for calculating a diagnostic score for the sample. In general, algorithms for use in determining diagnostic score for the sample can comprises using a SVM, logistic regression, lasso, boosting, bagging, random forest, CART, or MATT algorithm. Examples specific algorithm that may be applied to measurements of the markers disclosed herein include, but are not limited to, the following (u - indicates urine markers and p - indicates plasma markers):

Formula #1: : log_odds=1.1459+0.1776*sPSA-0.00004505*uPCA3-0.001314*pHSPD1+0.0001012*pIMPDH2+0.0006353*pPDLIM5-0.9314*pERG
odds=exp(log_odds)
prob=odds/(1+odds)

Formula #2: : log_odds=-0.1303+0. 786*sPSA+0.0000440*uPCA3-0.0013*pHSPD1+0.0000102*pIMPDH2+0.00000072856*pPDLIM5-0.00002379*pERG
odds=exp(log_odds)
prob=odds/(1+odds)

Formula #3: log_odds=0.1569+0.2786*sPSA-0.00004405*uPCA3-0.0001114*pHSPD1+0.0001052*pIMPDH2+0.0000006253*pPDLIM5-0.0009314*pERG
odds=exp(log_odds)
prob=odds/(1+odds)

Formula #5 : log_odds= 1.340e+00+1.999e-01*sPSA +1.237e-04*pERG -2.367e-05*uPDLIM5 + 1.613e-04*pUAP1
odds=exp(log_odds)
prob=odds/(1+odds)

Formula #5 : log_odds= -2.670e+00+2.955e-01*sPSA -2.288e-04*pERG - 7.885e-05*uPDLIM5 + 2.623e-04*pUAP1
odds=exp(log_odds)
prob=odds/(1+odds)

In some cases, after a proper functional form is determined, all expression markers in their proper functional form can be put together in a logistic regression equation. In addition to measuring the concordance index, the models can be examined for sensitivity and specificity. ROC (receiver operating characteristic) curves are graphed to examine the predictive ability of the models. ROC curves are simply a graph of a model's sensitivity vs. the false positive rate. The larger the area under the ROC curve (AUC), the better the model's concordance index and the better the model's ability at predicting recurrence with high sensitivity and specificity. AUC is simply the area that lies under the ROC curve; an AUC of 1 indicates perfect prediction ability - 100% sensitivity with 0% false positives. An AUC of 0.5 indicates that random chance is just as accurate at predicting outcome as the model. The closer the AUC is to 1, the better the predictive ability of the model. Concordance index is a measurement of the model's ability to distinguish risk, in other words that that low-risk observations are predicted to be of low probability and that observations at high risk for the event are predicted to occur with high probability. Sensitivity is the proportion of patients that tested positive for recurrence who actually later recurred. Specificity is the proportion of patients who tested negative for recurrence who actually did not recur. The false positive rate is 1 minus the specificity, in other words it is the proportion of patients who tested positive for recurrence but did not actually recur.

## II. Definitions

As used herein, "obtaining a biological sample" or "obtaining a blood sample" refer to receiving a biological or blood sample, *e.g.,* either directly or indirectly. Biological samples as used herein include essentially acellular body fluids, such as plasma, serum, and urine. For example, in some embodiments, the biological sample, such as a blood sample or a sample containing peripheral blood mononuclear cells (PBMC), is directly obtained from a subject at or near the laboratory or location where the biological sample will be analyzed. In other embodiments, the biological sample may be drawn or taken by a third party and then transferred, *e.g.,* to a separate entity or location for analysis. In other embodiments, the sample may be obtained and tested in the same location using a point-of care test. In these embodiments, said obtaining refers to receiving the sample, *e.g.,* from the patient, from a laboratory, from a doctor's office, from the mail, courier, or post office, *etc.* In some further aspects, the method may further comprise reporting the determination to the subject, a health care payer, an attending clinician, a pharmacist, a pharmacy benefits manager, or any person that the determination may be of interest.

By "subject" or "patient" is meant any single subject for which therapy or diagnostic test is desired. In this case the subjects or patients generally refer to humans. Also intended to be included as a subject are any subjects involved in clinical research trials not showing any clinical sign of disease, or subjects involved in epidemiological studies, or subjects used as controls.

As used herein, "increased expression" refers to an elevated or increased level of expression in a cancer sample relative to a suitable control (*e.g.*, a non-cancerous tissue or cell sample, a reference standard), wherein the elevation or increase in the level of gene expression is statistically significant ($p < 0.05$). Whether an increase in the expression of a gene in a cancer sample relative to a control is statistically significant can be determined using an appropriate t-test (*e.g.*, one-sample t-test, two-sample t-test, Welch's t-test) or other statistical test known to those of skill in the art. Genes that are overexpressed in a cancer can be, for example, genes that are known, or have been previously determined, to be overexpressed in a cancer.

As used herein, "decreased expression" refers to a reduced or decreased level of expression in a cancer sample relative to a suitable control (*e.g.,* a non-cancerous tissue or cell sample, a reference standard), wherein the reduction or decrease in the level of gene expression is statistically significant ($p < 0.05$). In some embodiments, the reduced or decreased level of gene expression can be a complete absence of gene expression, or an expression level of zero. Whether a decrease in the expression of a gene in a cancer sample relative to a control is statistically significant can be determined using an appropriate t-test (*e.g.,* one-sample t-test, two-sample t-test, Welch's t-test) or other statistical test known to those of skill in the art. Genes that are underexpressed in a cancer can be, for example, genes that are known, or have been previously determined, to be underexpressed in a cancer.

The term "antigen binding fragment" herein is used in the broadest sense and specifically covers intact monoclonal antibodies, polyclonal antibodies, multispecific antibodies (*e.g.,* bispecific antibodies) formed from at least two intact antibodies, and antibody fragments.

The term "primer," as used herein, is meant to encompass any nucleic acid that is capable of priming the synthesis of a nascent nucleic acid in a template-dependent process. Primers may be oligonucleotides from ten to twenty and/or thirty base pairs in length, but longer sequences can be employed. Primers may be provided in double-stranded and/or single-stranded form, although the single-stranded form is preferred.

### III. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice.

### Example 1 - Patients and Methods

*Patients and Samples.* Urine and blood samples were collected from 141 men that were classified into three groups. Arm 1 comprised 61 patients who were positive for prostate cancer after biopsy. Arm 2 comprised 60 patients who were negative for prostate cancer after biopsy. Arm 3 comprised 20 patients who recently underwent a prostatectomy. Histological grade of tumor per Gleason Score was provided for patients in Arm 1 and Arm 3. Serum PSA levels of each patient were measured and documented. Urine was collection from each patient without DRE, shipped immediately, and processed the following day. The volume of collected urine ranged from 30 mL to 110 mL. Each patient provided one collection cup with varying amounts of urine containing no preservatives and all patients provided approximately 9 mL of peripheral blood preserved in EDTA. All work was performed with an IRB-approved protocol (Western IRP) with consent form and all samples were collected from community practice urology groups.

*Urine and Plasma Processing.* Collected urine from each patient was concentrated by centrifugation using Amcion Ultra-15 Centrifugal Filter Units with 3 kDa membrane (Millipore, Billerica, Massachusetts). Urine was centrifuged using a swinging bucket rotor at 4,000 x g until only 1 mL of concentrated urine remained. Plasma was separated from peripheral blood samples and used for extraction of total nucleic acid. Total nucleic acid was extracted from patient urine and plasma using the NucliSens (BioMerieux, Durham, NC) extraction kit.

*Quantitative RT-PCR.* Quantitative RT-PCR was performed using the RNA Ultrasense One-Step Quantitative RT-PCR System (Applied Biosystems, Foster City, California) using a ViiA 7 Real-Time PCR System (Applied Biosystems) with the following thermocycler conditions: hold stage of 50°C for 15 min, 95°C for 2 min, followed by 45 cycles of 95°C for 15 seconds and 60°C for 30 seconds. The primer probe sets for PDLIM5, PCA3, TMPRSS2:ERG, and ERG were purchased as TaqMan® Gene Expression Assays with Assay IDs of Hs00935062_m1, Hs01371939_g1, Hs03063375, and Hs01554629_m1, respectively (Applied Biosystems). The primer probe set for UAP1 produced a PCR product of 70 bp: 5'-TTGCATTCAGAAAGGAGCAGACT-3' (forward; SEQ ID NO:1); 5'-CAACTGGTTCTGTAGGGTTCGTTT-3' (reverse; SEQ ID NO:2); and 5'-VIC®-TGGAGCAAAGGTGGTAGA-minor groove binder nonfluorescent quencher (MGBNNFQ)-3' (probe; SEQ ID NO:3). The primer probe set for HSPD1 produced a PCR product of 64 bp: 5'-AACCTGTGACCACCCCTGAA-3' (forward; SEQ ID NO:4); 5'-TCTTTGTCTCCGTTTGCAGAAA-3' (reverse; SEQ ID NO:5); 5'-VIC®ATTGCACAGGTTGCTAC-MGBNFQ-3' (probe; SEQ ID NO:6). The primer probe set for IMPDH2 was designed to encompass exons 10 and 11 and produced a PCR product of 74 bp: 5'-CCACAGTCATGATGGGCTCTC-3' (forward; SEQ ID NO:7); 5'-GGATCCCATCGGAAAAGAAGTA (reverse; SEQ ID NO:8); 5'-6FAM™-ACCACTGAGGCCCCT-MGBNFQ-3' (probe; SEQ ID NO:9). The primer probe set for PSA produced a PCR product of 67 bp: 5'-CCACTGCATCAGGAACAAAG-3' (forward; SEQ ID NO:10); 5-TGTGTCTTCAGGATGAAACAGG-3' (reverse; SEQ ID NO:11); 5'-VIC®-CGTGATCTTGCTGGGT-MGBNNFQ (probe; SEQ ID NO:12). B2M and GAPDH mRNA transcripts were measured as controls and purchased as Pre-Developed TaqMan® Assay Reagents (Applied Biosystems). Human prostate carcinoma cells (CRL-2505) were used to provide RNA for positive control (ATCC) and extracted with QIAamp RNA Blood Mini Kit (Qiagen, Hilden, Germany). Negative controls were obtained from First Choice® Human Prostate Total RNA (Applied Biosystems).

## Example 2 - Results

Patients Characteristics. Patients with biopsy-confirmed prostate cancer and BPH were of similar age (median 66 vs. 63, respectively) (p = 0.21) (Table 1). Ethnic distribution was also similar with the majority of patients being white (Table 1). However, as expected there was a significant difference between the two groups in serum PSA (p < 0.001), with a median of 4.4 ng/ml in the BPH group and 5.7 ng/ml in the cancer group (Table 1). As a control data and samples were collected on 20 patients after prostatectomy for prostate cancer. As shown in Table 1, this group of patients had similar age and ethnic background, but PSA was also significantly lower than both BPH and cancer groups (median of 0.01 ng/ml). Gleason histologic grade was similar between the cancer patients and post-prostatectomy patients. Gleason grading was performed according to the new modified system based on the 2005 consensus conference.

Significant Difference Between Post-Prostatectomy and Both Cancer and BPH Patients. In univariate analysis, there were significant (p < 0.05) differences between the post-prostatectomy patients and cancer group in PDLIM5 (p = 0.005), UAP1 (p = 0.001), PCA3 (p < 0.0001), TMPRSS (p = 0.009) in urine and HSPD (p = 0.01), IMPDH2 (p = 0.003), UAP1 (p = 0.02), and ERG (p = 0.02) in plasma.

There was a significant difference between post-prostatectomy and BPH in HSPD1 (p = 0.004), IMPDH2 (p = 0.002), PDLMI5 (p = 0.0003), UAP1 (p = 0.0003), PCA3 (p < 0.0001), TMPRSS and (p = 0.0006) in urine and HSPD (p = 0.006), IMPDH2 (p = 0.002), UAP1 (p = 0.03) in plasma. This clearly shows that most of these markers are prostate-specific and this is reflected in plasma samples as well as urine samples.

Marginal Difference Between BPH and Prostate Cancer using Univariate Comparison. In univariate analysis, there were significant differences between BPH and prostate cancer only in HSPD1 (p = 0.05), IMPDH2 (p = 0.01), PDLIM5 (p = 0.05) in urine and Erg (p = 0.0003) in plasma.

Except for plasma ERG expression, the differences between BPH and cancer were minimal, which reflects the difficulty in distinguishing between the two conditions and most likely is due to the fact most patients with cancer also have BPH.

Multivariate Analysis and the Development of an Algorithm to Distinguish Cancer from BPH. In order to be able to distinguish patients with prostate cancer from BPH and at the same time take advantage of as many variables as possible, but also eliminate variables that are not relevant, the inventors explored the value of mathematical algorithms. The inventors first divided the samples into a learning (training) group, which included 70 patients (35 cancer and 35 BPH), and a testing group, which included 51 patients (26 cancer and 25 BPH). Furthermore, the training set was also used with approximately two third for model creation and one third for testing before validation of the model using the testing 51 patients set. The variables included in developing the algorithm were UAP1, PDLIM5, IMPDH2, HSPD1, PCA3, PSA, TMPRSS2, ERG, GAPDH, B2M, age and serum PSA.

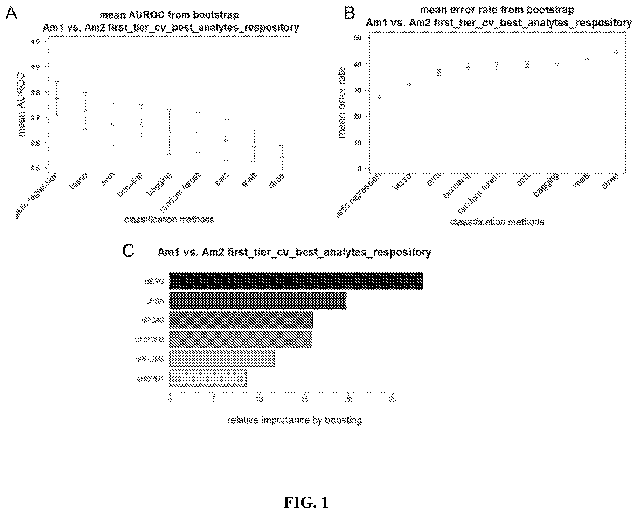

The inventors used multiple mathematical algorithms for features selection and compared the mean AUC and the mean error rates between various algorithms. All used algorithms were based on machine learning and included logistic regression, SVM (Support vector machine), Lasso (least absolute shrinkage and selection operator), boosting, bagging, random forest, CART (classification and regression tree), matt, and ctree (Conditional interference tree). As shown in Table 2 and FIG. 1, the best AUC and the least error rate from all algorithms was obtained by logistic regression. In this algorithm testing of the training set showed AUC of 0.77 and mean error rate of 0.27. In this model, six variables were included and the contribution of each variable is shown in FIG. 1. Feature elimination was used to eliminate variables that were not contributing to improve the model. The six variables included in this model were plasma ERG, serum PSA, urine PCA3, urine MPDH2, urine PDLIM5, and urine HSPD1.

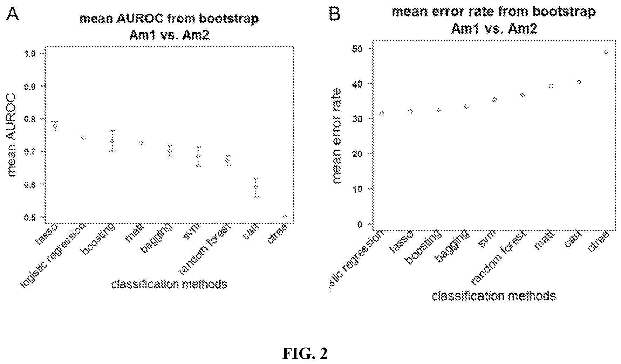

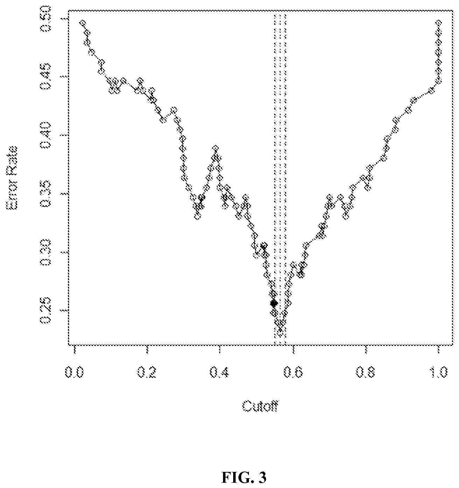

When the same model was applied to the test set, similar results were obtained (FIG. 2). For logistic regression, the inventors obtained a mean AUC value of 0.78 for this set. When all 121 samples were considered and each group was tested 100 times selecting random samples each time, the inventors obtained AUCs that varied between 0.70 and 0.85. The logistic regression algorithm suggested a cut-off point of 0.565 (FIG. 3) with a least error rate of 0.25. At this cut-off point, the specificity and sensitivity are at 88% and 67%, respectively.

In this group of patients using serum PSA alone and cut off point of 4, the specificity was at 62% and sensitivity at 56%. Using sPSA cutoff >14.1, we obtain 100% specificity but 18% sensitivity.

Multivariate Analysis and the Development of an Algorithm to Distinguish Aggressive Prostate Cancer. It has been suggested that in the modified Gleason scoring system Score <7 is indolent cancer and the risk of mortality from the cancer is very small. In patients with prostate cancer Gleason score<6, the risk of dying within 10 to 15 years post diagnosis is the same whether treated or not (Carter et al, JCO, Dec 10, 2012). Therefore, we lumped patients with Gleason <7 along with patients with BPH and explored the potential of our biomarkers in predicting the prostate cancer patients with Gleason ≥7 (32 patients) from the rest of the patients (Gleason <7 and BPH) (89 patients).

The whole data set was partitioned randomly into training (69 patients) including 18 patients with aggressive cancer and 51 with BPH/Gleason<7. The testing group (52 patients) included 14 patients with aggressive cancer and 38 patients with BPH/Gleason<7.

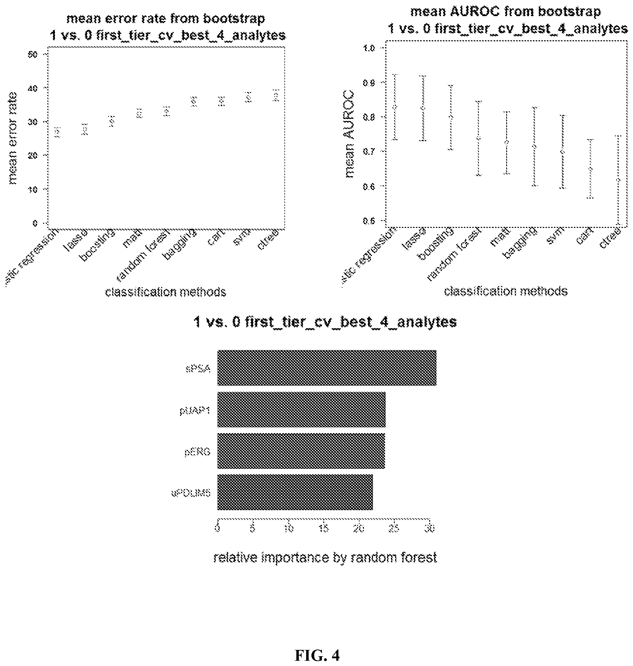

Mathematical models were created in the same fashion as described above using training set and AUC and error rates were compared. FIG. 4 shows the mean AUC and the error rate for each of the algorithms. Again logistic regression showed the most informative model with a mean AUC of 0.87 in the training set based on testing 100 times after random selection. The testing set showed AUC of 0.88. When all samples were combined and tested, the AUC was 0.88. In this model, four variables were adequate for developing this algorithm and this included serum PSA, plasma UPA1, plasma ERG and urine PDCIM5 as shown in FIG. 4. The contribution of each of these variables is shown in FIG. 4C.

Based on AUC, we selected 0.61 as a cut-off, which gives specificity of 0.99 and sensitivity of 0.47 (Table 3).

The number of advanced cancer is relatively small (32 patients), however, the AUC value of 0.87 is within one standard deviation. The mean ± 1SD was 0.73 to 0.92 based on 50 iteration testing.

Combined Model for Detecting Patients with Aggressive Cancer from Patients with Indolent Cancer or BPH. The two models described above are completely independent using different variables and different algorithms. When an individual patient is evaluated using both models, obtaining concordant results by the two models most likely represent stronger prediction. To investigate this the inventors compared results between the two models using all 121 patients. Of the 121 patients, 91 (75%) had concordant results. In this group of patients, specificity and sensitivity was 99% and 68%, respectively, in predicting aggressive cancer vs. indolent cancer or BPH (Table 4, FIG. 6). The rest of the patients (25% of total number) had discordant results and for practical reasons should be considered only in predicting the presence or absence of prostate cancer with a specificity and sensitivity of 88% and 67%, but cannot be reliably classified for the aggressiveness of the cancer.

**Table 1.** Characteristics of patients used in the study.

|  | Cancer | BPH | Post-Pros | P-Value |
|---|---|---|---|---|
| **Age [Median (range)** | 66 (45-84) | 63 (45-84) | 67 (50-77) | 0.21 |
| **Race** | 82%W, 5%B, 10%H, 2%A | 78%W, 3%B, 17%H, 0%A | 85%W, 5%B, 10%H, 0%A | 0.73 |
| **Histologic grade** | 47%(1), 23%(2), 15%(3), 15%(4) |  | 21%(1), 53%(2), 16%(3), 10%(4) | 0.26 |
| **PSA (ng/ml)** | 5.7 (1.5-283) | 4.4 (0.5-14.1) | 0.01 (0-6.0) | <0.001 |

**Table 2.** The AUCs and error rates obtained by various mathematical algorithms to distinguish between cancer and BPH using a training set.

| Method | Mean-AUROC | std-AUROC | Mean-err | std-err |
|---|---|---|---|---|
| **logistic regression** | 0.773 | 0.067 | 0.269 | 0.01 |
| **lasso** | 0.726 | 0.072 | 0.322 | 0.01 |
| **svm** | 0.672 | 0.082 | 0.365 | 0.012 |

(continued)

| Method | Mean-AUROC | std-AUROC | Mean-err | std-err |
|---|---|---|---|---|
| boosting | 0.667 | 0.084 | 0.387 | 0.01 |
| bagging | 0.643 | 0.089 | 0.392 | 0.012 |
| random forest | 0.642 | 0.079 | 0.397 | 0.011 |
| cart | 0.609 | 0.081 | 0.397 | 0.01 |
| matt | 0.586 | 0.061 | 0.415 | 0.008 |
| ctree | 0.54 | 0.049 | 0.444 | 0.006 |

**Table 3.** Mean AUC and the standard deviation for distinguishing aggressive prostate cancer from BPH/indolent.

| Method | mean_AUROC | std_AUROC |
|---|---|---|
| logistic regression | 0.828 | 0.094 |
| Lasso | 0.824 | 0.094 |
| boosting | 0.797 | 0.093 |
| random forest | 0.738 | 0.107 |
| Matt | 0.725 | 0.089 |
| Bagging | 0.713 | 0.113 |
| Svm | 0.699 | 0.105 |
| Cart | 0.649 | 0.084 |
| Ctree | 0.617 | 0.128 |

**Table 4.** Sensitivity, specificity, positive predictive (PPV) and negative predictive value (NPV) for the three algorithms.

| | | Estimated Value | 95% Confidence | |
|---|---|---|---|---|
| | | | Lower Limit | Upper Limit |
| Cancer Vs. BPH at cut-off = 0.565 | Sensitivity | 0.67 | 0.54 | 0.78 |
| | Specificity | 0.88 | 0.77 | 0.95 |
| | PPV | 0.85 | 0.72 | 0.93 |
| | NPV | 0.73 | 0.61 | 0.82 |
| Aggressive Cancer Vs. BPH/Gleason<7 at cut-off = 0.61 | Sensitivity | 0.47 | 0.30 | 0.65 |
| | Specificity | 0.99 | 0.93 | 1.00 |
| | PPV | 0.94 | 0.68 | 1.00 |
| | NPV | 0.84 | 0.75 | 0.90 |
| Combined model for predicting Aggressive Cancer Vs. BPH/Gleason<7 | Sensitivity | 0.68 | 0.45 | 0.85 |
| | Specificity | 0.99 | 0.91 | 1.00 |
| | PPV | 0.94 | 0.68 | 1.00 |
| | NPV | 0.91 | 0.81 | 0.96 |

**Example 3 - Assays using additional markers**

*Materials and methods*

*Study Design and Patients*

Urine and blood samples from 287 men presenting with prostate enlargement and scheduled for prostate biopsies from four urology practices were collected. Histologic GS of tumors for biopsy confirmed PCa was provided by the sites for each patient. Gleason grading was performed according to the new modified system based on the 2005 consensus conference (Epstein *et al.* 2006, incorporated herein by reference). Biopsies showed that 103 (36%) of patients had BPH and 184 (64%) patients had PCa. 107 of the PCa patients were in the high risk group (58% of PCa and 37% of the total). Patients receiving any therapy for BPH or PCa were excluded and patients were required to be newly diagnosed in order to participate in the study. Urine samples were collected without digital rectal exam (DRE) and were processed within 48 hours of collection. 9 mL of peripheral blood in ethylenediaminetetraacetic acid (EDTA) was provided by all patients. There were no other selection criteria, samples represent average patients. All labwork was performed with the IRB-approved protocol (Western IRP).

*Urine and Plasma Processing*

Voided urine from each patient was concentrated to a volume of 1 ml by centrifugation using the Amcion Ultra-15 Centrifugal Filter Unit with a 3KDa membrane (Millipore, Billerica, MA) in a swinging bucket rotor at 4,000 x g. Plasma was separated from peripheral blood using standard centrifugation. Total nucleic acid was extracted from concentrated urine or plasma using the NucliSENS® extraction kit (BioMerieux, Durham, NC).

*Quantitative Reverse Transcription-Polymerase Chain Reaction (qRT-PCR)*

Quantitative reverse transcription-real-time polymerase chain reaction (qRT-PCR) was performed using the RNA Ultrasense One-Step Quantitative RT-PCR System (Applied Biosystems, Foster City, CA) on a ViiA™ 7 Real-Time PCR System (Applied Biosystems) with the following thermocycler conditions: hold stage of 50°C for 15 min, 95°C for 2 min, followed by 45 cycles of 95°C for 15 seconds and 60°C for 30 seconds. Six-point serial dilution standards were obtained from First Choice® Human Prostate Total RNA (Applied Biosystems). The PDLIM5, PCA3, TMPRSS2, ERG and PTEN primers and probes were purchased as TaqMan Gene Expression Assays with assay IDs of Hs00935062_m1, Hs01371939_g1, Hs01120965m1, Hs01554629_m1, and Hs01920652_s1, respectively (Applied Biosystems). The primer probe set for UAP1 produced a PCR product of 70bp: 5'-TTGCATTCAGAAAGGAGCAGACT-3' (forward; SEQ ID NO:1); 5'-CAACTGGTTCTGTAGGGTTCGTTT-3' (reverse; SEQ ID NO:2); and VIC®-TGGAGCAAAGGTGGTAGA-MGBNFQ (probe; SEQ ID NO:3). The primer probe set for HSPD1 produced a PCR product of 64bp: 5'-AACCTGTGACCACCCCTGAA-3' (forward; SEQ ID NO:4); 5'-TCTTTGTCTCCGTTTGCAGAAA-3' (reverse; SEQ ID NO:5); VIC®-ATTGCACAGGTTGCTAC-MGBNFQ (probe; SEQ ID NO:6). The primer probe set for IMPDH2 was designed to encompass exons 10 and 11 and produced a PCR product of 74bp: 5'-CCACAGTCATGATGGGCTCTC-3' (forward; SEQ ID NO:7); 5'-GGATCCCATCGGAAAAGAAGTA (reverse; SEQ ID NO:8); 6FAM™-ACCACTGAGGCCCCT-MGBNFQ (probe; SEQ ID NO:9). The primer probe set for PSA produced a PCR product of 67bp: 5'-CCACTGCATCAGGAACAAAAG-3' (forward; SEQ ID NO:10); 5'-TGTGTCTTCAGGATGAAACAGG-3' (reverse; SEQ ID NO:11); VIC®-CGTGATCTTGCTGGGT-MGBNNFQ (probe; SEQ ID NO:12).The primer probe set for AR was designed to encompass exons 6 and 7 and produced a PCR product of 91bp: 5'-GGAATTCCTGTGCATGAAAGC-3' (forward; SEQ ID NO:13); 5'-CATTCGAAGTTCATCAAAGAATT-3' (reverse; SEQ ID NO:14); VIC® - CTTCAGCATTATTCCAGTG-MGBNFQ (probe; SEQ ID NO:15). Pre-Developed TaqMan® Assay Reagents (Applied Biosystems) for B2M and GAPDH were purchased in order to measure their mRNA transcripts as controls. In all assays, an equal amount of plasma was used for RNA extraction, RNA was eluted into an equal amount of elution buffer, and an equal amount of RNA solution was used in each assay. Similarly, for urine, RNA was extracted from 1ml of total concentrate urine, eluted into an equal amount of elution buffer, and an equal amount of RNA solution was used in each assay.

*Results*

Biopsy results showed that 103 (36%) of the 287 patients had BPH and 184 (64%) patients had PCa, of which 107 (58% of PCa and 37% of total) had high-risk PCa. Using the training set, algorithms were developed for distinguishing PCa from BPH. For this assessment the markers used were (1) serum PSA protein level; (2) plasma ERG mRNA level; (3) plasma AR mRNA level; (4) urine PCA3 mRNA level; (5) urine PTEN level; (6) urine B2M mRNA level; (7) plasma B2M mRNA level; and (8) plasma GAPDH mRNA level. Using these markers PCa could be distinguished from BPH with area under the receiver operating characteristic curve (AUROC) of 0.87. The testing set for this model showed sensitivity of 76% and specificity of 71% upon using a cut-off point of 0.64 (see, *e.g.,* FIG. 7 and Table 5).

**Table 5:** Results from testing set in predicting PCa at 0.64 cut-off

| | | 95% Confidence Interval | |
|---|---|---|---|
| Estimated Value | | Lower Limit | Upper Limit |
| Prevalence | 0.65 | 0.55 | 0.74 |
| Sensitivity | 0.76 | 0.64 | 0.86 |
| Specificity | 0.71 | 0.52 | 0.84 |
| For any particular test result, the probability that it will be: | | | |
| Positive | 0.60 | 0.49 | 0.69 |
| Negative | 0.40 | 0.31 | 0.51 |
| For any particular positive test result, the probability that it is: | | | |
| True Positive | 0.83 | 0.70 | 0.91 |
| False Positive | 0.17 | 0.09 | 0.30 |
| For any particular negative test result, the probability that it is: | | | |
| True Negative | 0.62 | 0.45 | 0.76 |
| False Negative | 0.38 | 0.24 | 0.55 |

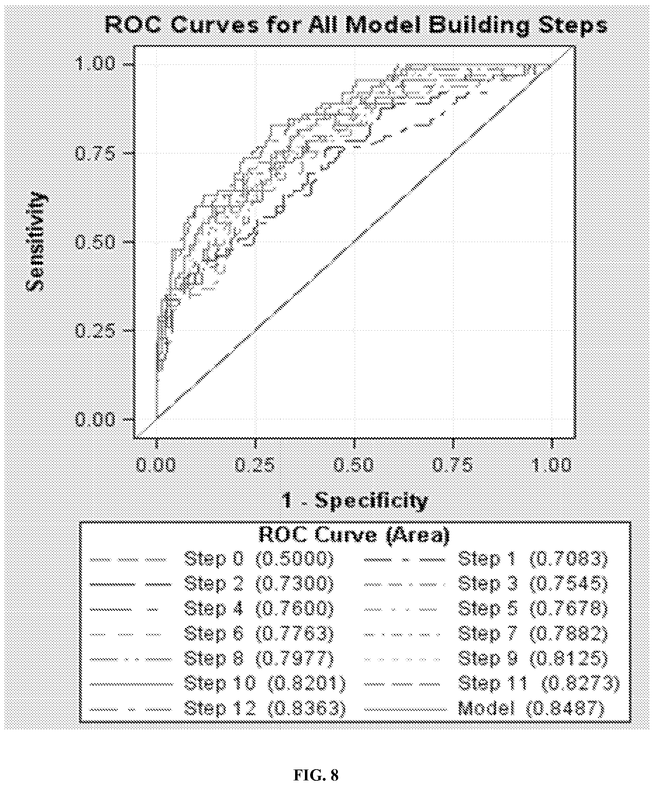

Additional algorithms were developed for predicting patients with high-risk PCa (GS ≥7) vs. GS <7 cancer or BPH. For this assessment the markers used were (1) serum PSA protein level; (2) Age; (3) urine PSA mRNA level; (4) plasma ERG mRNA level; (5) urine GAPDH mRNA level; (6) urine B2M mRNA level; (7) urine PTEN mRNA level; (8) urine PCA3 mRNA level; and (9) urine PDLIM5 mRNA level. With these markers high-risk PCa could be distinguished from low-grade cancer (GS <7) or BPH with an AUROC of 0.80 (see, *e.g.,* FIG. 8 and Table 6). In some further calculations an additional three markers ((10) plasma PCA3 mRNA level; (11) plasma B2M mRNA level and (12) plasma HSPD1 mRNA level) were used, which achieved an AUROC of 0.8487.

**Table 6:** Results from testing set in predicting high-risk PCa at 0.27 cut-off

| | | 95% Confidence Interval | |
|---|---|---|---|
| Estimated Value | | Lower Limit | Upper Limit |
| Prevalence | 0.35 | 0.26 | 0.44 |
| Sensitivity | 0.44 | 0.28 | 0.60 |
| Specificity | 0.76 | 0.64 | 0.85 |
| For any particular test result, the probability that it will be: | | | |
| Positive | 0.31 | 0.23 | 0.40 |
| Negative | 0.69 | 0.60 | 0.77 |
| For any particular positive test result, the probability that it is: | | | |
| True Positive | 0.49 | 0.32 | 0.66 |
| False Positive | 0.51 | 0.34 | 0.68 |
| For any particular negative test result, the probability that it is: | | | |
| True Negative | 0.72 | 0.60 | 0.81 |
| False Negative | 0.28 | 0.19 | 0.40 |

Further analysis showed that patients with concordant results between the two analyses showed specificity of 89% and sensitivity of 59% for having high-grade aggressive PCa (Table 7), and specificity of 94% and sensitivity of 81% for having PCa and not BPH (Table 8), but with tolerating the non-detection of low-risk PCa. Thus, combining the two analyses and accepting a diagnosis of PCa if one of the two was positive for cancer, regardless of the aggressiveness, showed specificity and sensitivity of 82% and 92% respectively (Table 9), with the possibility of missing low-risk cancer (PPV=86% and NPV=90%). Biomarkers making the strongest contributions in both algorithms were plasma and urine ERG, PTEN, AR, and PCA3 mRNAs in addition to the sPSA, and to a lesser degree, PDLIM5 and PSA mRNA in plasma and urine.

**Table 7:** Combined analyses for detecting high-grade aggressive PCa (Both analyses positive or negative: 184 of 287, 64%)

| | 95% Confidence Interval | | |
|---|---|---|---|
| Estimated Value | | Lower Limit | Upper Limit |
| Prevalence | 0.35 | 0.28 | 0.42 |
| Sensitivity | 0.59 | 0.46 | 0.71 |
| Specificity | 0.89 | 0.82 | 0.94 |
| For any particular test result, the probability that it will be: | | | |
| Positive | 0.28 | 0.22 | 0.35 |
| Negative | 0.72 | 0.65 | 0.78 |
| For any particular positive test result, the probability that it is: | | | |
| True Positive | 0.75 | 0.60 | 0.85 |
| False Positive | 0.25 | 0.15 | 0.40 |
| For any particular negative test result, the probability that it is: | | | |
| True Negative | 0.80 | 0.72 | 0.87 |
| False Negative | 0.20 | 0.13 | 0.28 |

**Table 8:** Concordant results for detecting PCa and not BPH accepting that cancer, if GS <7 is tolerated if either missed or detected (184 of 287, 64%).

| | 95% Confidence Interval | | |
|---|---|---|---|
| Estimated Value | | Lower Limit | Upper Limit |
| Prevalence | 0.38 | 0.31 | 0.46 |
| Sensitivity | 0.81 | 0.70 | 0.89 |
| Specificity | 0.94 | 0.87 | 0.97 |
| For any particular test result, the probability that it will be: | | | |
| Positive | 0.35 | 0.28 | 0.42 |
| Negative | 0.65 | 0.58 | 0.72 |
| For any particular positive test result, the probability that it is: | | | |
| True Positive | 0.89 | 0.78 | 0.95 |
| False Positive | 0.11 | 0.05 | 0.22 |
| For any particular negative test result, the probability that it is: | | | |
| True Negative | 0.89 | 0.82 | 0.94 |
| False Negative | 0.11 | 0.06 | 0.18 |

**Table 9:** Results if either analysis is positive for PCa, regardless of the aggressiveness, and assuming GS <7 is tolerated if determined as negative.

| Estimated Value | | 95% Confidence Interval | |
|---|---|---|---|
| | | Lower Limit | Upper Limit |
| Prevalence | 0.54 | 0.48 | 0.60 |
| Sensitivity | 0.92 | 0.86 | 0.95 |
| Specificity | 0.82 | 0.74 | 0.88 |
| For any particular test result, the probability that it will be: | | | |
| Positive | 0.58 | 0.52 | 0.64 |
| Negative | 0.42 | 0.36 | 0.48 |
| For any particular positive test result, the probability that it is: | | | |
| True Positive | 0.86 | 0.79 | 0.90 |
| False Positive | 0.14 | 0.10 | 0.21 |
| For any particular negative test result, the probability that it is: | | | |
| True Negative | 0.89 | 0.82 | 0.94 |
| False Negative | 0.11 | 0.06 | 0.18 |

Thus, by combining the results of the two analysis described supra (*i.e.,* assay of markers for distinguishing PCa from BPH and assay of marker for distinguishing high-risk PCa from low risk PCa (GS < 7) or BPH) a highly specific and sensitive diagnosis can be achieved. Specific diagnostic results achieved with the studies detailed here indicate:

1) Both analyses negative:

- No evidence of any prostate cancer (Sens=59%, Spec=89%)

- No evidence of high-risk aggressive (Gleason ≥7), but cannot fully rule out Low grade Cancer (Gleason<7) (Sens=81%, Spec=94%)

2) Both analyses positives:

- High-probability of having aggressive cancer (Gleason >7) (Sens=59%, Spec=89%)

- High probability of having any prostate cancer (any grade) (Sens=81%, Spec=94%)

3) PCa positive and High-grade negative:

- High probability of having any cancer (Sens=92%, Spec=82), but unlikely to be high grade (Spec=76%, Sens=44%)

4) PCa negative and High grade positive

- High probability of having any cancer (Sens=92%, Spec=82), but likely to be high grade (Spec=76%, Sens=44%)

All of the methods disclosed and claimed herein can be made and executed without undue experimentation in light of the present disclosure. While the compositions and methods of this invention have been described in terms of preferred embodiments, it will be apparent to those of skill in the art that variations may be applied to the methods and in the steps or in the sequence of steps of the method described herein. More specifically, it will be apparent that certain agents which are both chemically and physiologically related may be substituted for the agents described herein while the same or similar results would be achieved.

## REFERENCES

Ausubel et al., Current protocols in molecular biology, John Wiley & Sons Ltd, Wiley Interscience, 2003.
Carter et al., J. Clin. Oncol., 30:4294-4296, 2012.
Epstein et al., "Update on the Gleason grading system for prostate cancer: results of an international consensus conference of urologic pathologists," Adv. Anat. Pathol., 13(I):57-9, 2006.
Sambrook et al., Molecular cloning: A laboratory manual, Cold Spring Harbor Laboratory Press, 1989.
Prior et al., "Use of a combination of biomarkers in serum and urine to improve detection of prostate cancer", World Journal of Urology, Springer, Berlin, DE, Vol. 28, No. 6, 681-686, 2010.
EP 2 373 816 A2.
WO 2012/115885 A1.
Prensner et al., "Beyond PSA: The Next Generation of Prostate Cancer Biomarkers", Science Translational Medicine, American Association of the Advancement of Science, Washington, DC, Vol. 4, No. 127, 54-64, 2012.
Guyon et al., "A Four-Gene Expression Signature for Prostate Cancer Cells Consisting of UAP1, PDLIM5, IMPDH2, and HSPD1", Urotoday International Journal, Vol. 02, No. 04, 1944-5784 (2009).
WO 2010/065940 A1.
WO 2006/056766 A2.
WO 2005/113816 A2.
WO 2012/092336 A2.

## Claims

1. An *in vitro* method of detecting if a subject is at risk for prostate cancer or aggressive prostate cancer, comprising:

   (a) obtaining a blood sample and urine sample from the subject;
   (b) measuring the mRNA expression level of a set of the blood and urine markers consisting of the UAP1, PDLIM5, IMPDH2, HSPD1, PCA3, PSA, TMPRSS2, ERG, GAPDH, B2M, PTEN and AR genes from the urine sample and from the blood sample; and
   (c) identifying the subject as at risk or not at risk for prostate cancer or aggressive prostate cancer based on the expression level of said genes.

2. The method of claim 1, wherein identifying the subject as at risk or not at risk for prostate cancer or aggressive prostate cancer further comprises:

   (i) correlating the expression levels of said genes with a risk for prostate cancer or aggressive prostate cancer; or
   (ii) analysis of the expression levels of said genes using a SVM, logistic regression, lasso, boosting, bagging, random forest, CART, or MATT algorithm.

3. The method of claim 2, wherein said correlating is performed by a computer.

4. The method of claim 1, wherein the subject:

   (i) has previously had a prostatectomy;
   (ii) has or is diagnosed with an enlarged prostate or benign prostate hyperplasia (BPH).

5. The method of claim 1, further comprising:

in (b) measuring the expression level of the genes in the sample and measuring the expression level of the genes in a reference sample; and

in (d) identifying the subject as at risk or not at risk for prostate cancer or aggressive prostate cancer by comparing the expression level of the genes in the sample from the subject to the expression level of the genes in the reference sample.

6. The method of claim 1, wherein measuring expression in said genes comprises measuring protein expression levels.

7. The method of claim 6, wherein measuring protein expression levels comprises performing an ELISA.

8. The method of claim 1, wherein the measuring expression in said genes comprises measuring RNA expression levels.

9. The method of claim 8, wherein measuring RNA expression levels comprises performing RT-PCR, Northern blot or an array hybridization.

10. The method of claim 1, further comprising reporting whether the subject has a prostate cancer or has an aggressive prostate cancer, such as by preparing a written or electronic report.

11. A tangible computer-readable medium comprising computer-readable code that, when executed by a computer, causes the computer to perform operations comprising:

a) receiving information corresponding to a level of expression of the UAP1, PDLIM5, IMPDH2, HSPD1, PCA3, PSA, TMPRSS2, ERG, GAPDH, B2M, PTEN and AR genes in a sample from a subject; and

b) determining a relative level of expression of said genes compared to a reference level, wherein altered expression of said genes compared to a reference level indicates that the subject is at risk of having prostate cancer or aggressive prostate cancer.

**Patentansprüche**

1. In-vitro-Verfahren zum Detektieren, ob ein Subjekt von Prostatakrebs oder aggressivem Prostatakrebs bedroht ist, umfassend:

(a) Erhalten einer Blutprobe und Urinprobe von dem Subjekt;

(b) Messen des mRNA-Expressionslevels von einem Set aus den Blut- und Urin-Markern bestehend aus den UAP1-, PDLIM5-, IMPDH2-, HSPD1-, PCA3-, PSA-, TMPRSS2-, ERG-, GAPDH-, B2M-, PTEN- und AR-Genen aus der Urinprobe und aus der Blutprobe; und

(c) Identifizieren des Subjekts als bedroht oder nicht bedroht von Prostatakrebs oder aggressivem Prostatakrebs basierend auf dem Expressionslevel dieser Gene.

2. Verfahren nach Anspruch 1, wobei Identifizieren des Subjekts als bedroht oder nicht bedroht von Prostatakrebs oder aggressivem Prostatakrebs weiter umfasst:

(i) Korrelieren der Expressionslevel dieser Gene mit einem Risiko für Prostatakrebs oder aggressiven Prostatakrebs; oder

(ii) Analyse der Expressionslevel dieser Gene unter Verwenden eines SVM-, logistischen Regressions- (logistic regression), Lasso-, Boosting-, Bagging-, Random-Forest-, CART- oder MATT-Algorithmus.

3. Verfahren nach Anspruch 2, wobei dieses Korrelieren von einem Computer durchgeführt wird.

4. Verfahren nach Anspruch 1, wobei das Subjekt:

(i) zuvor eine Prostatektomie hatte;

(ii) eine vergrößerte Prostata oder eine gutartige Prostatahyperplasie (BPH) aufweist oder damit diagnostiziert wurde.

**5.** Verfahren nach Anspruch 1, weiter umfassend:

in (b) Messen des Expressionslevels der Gene in der Probe und Messen des Expressionslevels der Gene in einer Referenzprobe; und

in (d) Identifizieren des Subjekts als bedroht oder nicht bedroht von Prostatakrebs oder aggressivem Prostatakrebs durch Vergleichen des Expressionslevels der Gene in der Probe von dem Subjekt mit dem Expressionslevel der Gene in der Referenzprobe.

**6.** Verfahren nach Anspruch 1, wobei Messen der Expression dieser Gene Messen der Proteinexpressionslevel umfasst.

**7.** Verfahren nach Anspruch 6, wobei Messen der Proteinexpressionslevel Durchführen eines ELISA umfasst.

**8.** Verfahren nach Anspruch 1, wobei das Messen der Expression in diesen Genen Messen der RNA-Expressionslevel umfasst.

**9.** Verfahren nach Anspruch 8, wobei Messen der RNA-Expressionslevel Durchführen von RT-PCR, Northern-Blot oder einer Arrayhybridisierung umfasst.

**10.** Verfahren nach Anspruch 1, weiter umfassend Berichten, ob das Subjekt einen Prostatakrebs aufweist oder einen aggressiven Prostatakrebs aufweist, wie durch Vorbereiten eines schriftlichen oder elektronischen Berichts.

**11.** Greifbares computerlesbares Medium, umfassend computerlesbaren Code, der, wenn von einem Computer ausgeführt, den Computer veranlasst, Operationen durchzuführen, umfassend:

a) Erhalten von Information entsprechend einem Expressionslevel der UAP1-, PDLIM5-, IMPDH2-, HSPD1-, PCA3-, PSA-, TMPRSS2-, ERG-, GAPDH-, B2M-, PTEN- und AR-Gene in einer Probe von einem Subjekt; und
b) Bestimmen eines relativen Expressionslevels dieser Gene verglichen mit einem Referenzlevel, wobei veränderte Expression dieser Gene verglichen mit einem Referenzlevel anzeigt, dass das Subjekt bedroht ist, Prostatakrebs oder aggressiven Prostatakrebs aufzuweisen.

**Revendications**

**1.** Procédé *in vitro* pour détecter si un sujet est à risque pour le cancer de la prostate ou le cancer de la prostate agressif, comprenant les étapes consistant à :

(a) obtenir un échantillon de sang et un échantillon d'urine du sujet ;
(b) mesurer le niveau d'expression d'ARNm d'un ensemble des marqueurs du sang et de l'urine constitué par les gènes UAP1, PDLIM5, IMPDH2, HSPD1, PCA3, PSA, TMPRSS2, ERG, GAPDH, B2M, PTEN et AR de l'échantillon d'urine et de l'échantillon de sang ; et
(c) identifier le sujet comme à risque ou non à risque pour le cancer de la prostate ou le cancer de la prostate agressif sur la base du niveau d'expression desdits gènes.

**2.** Procédé selon la revendication 1, dans lequel l'identification du sujet comme à risque ou non à risque pour le cancer de la prostate ou le cancer de la prostate agressif comprend en outre :

(i) la corrélation des niveaux d'expression desdits gènes avec un risque pour le cancer de la prostate ou le cancer de la prostate agressif ; ou
(ii) l'analyse des niveaux d'expression desdits gènes en utilisant un algorithme SVM, de régression logistique, lasso, boosting, bagging, random forest, CART ou MATT.

**3.** Procédé selon la revendication 2, dans lequel ladite corrélation est effectuée par ordinateur.

**4.** Procédé selon la revendication 1, dans lequel le sujet :

(i) a eu précédemment une prostatectomie ;
(ii) a ou est diagnostiqué avec une prostate agrandie ou une hyperplasie prostatique bénigne (BPH).

**5.** Procédé selon la revendication 1, comprenant en outre les étapes consistant à :

dans (b) mesurer le niveau d'expression des gènes dans l'échantillon et mesurer le niveau d'expression des gènes dans un échantillon de référence ; et

dans (d) identifier le sujet comme à risque ou non à risque pour le cancer de la prostate ou le cancer de la prostate agressif en comparant le niveau d'expression des gènes dans l'échantillon du sujet au niveau d'expression des gènes dans l'échantillon de référence.

**6.** Procédé selon la revendication 1, dans lequel la mesure de l'expression dans lesdits gènes comprend la mesure des niveaux d'expression de protéine.

**7.** Procédé selon la revendication 6, dans lequel la mesure des niveaux d'expression de protéine consiste à effectuer un ELISA.

**8.** Procédé selon la revendication 1, dans lequel la mesure de l'expression dans lesdits gènes comprend la mesure des niveaux d'expression d'ARN.

**9.** Procédé selon la revendication 8, dans lequel la mesure des niveaux d'expression d'ARN consiste à effectuer une RT-PCR, un transfert de Northern ou une hybridation sur puce.

**10.** Procédé selon la revendication 1, comprenant en outre l'étape consistant à rapporter si le sujet a un cancer de la prostate ou a un cancer de la prostate agressif, tel qu'en préparant un rapport écrit ou électronique.

**11.** Support lisible sur ordinateur tangible comprenant un code lisible sur ordinateur qui, quand il est exécuté par un ordinateur, amène l'ordinateur à effectuer les opérations comprenant :

a) la réception des informations correspondant à un niveau d'expression des gènes UAP1, PDLIM5, IMPDH2, HSPD1, PCA3, PSA, TMPRSS2, ERG, GAPDH, B2M, PTEN et AR dans un échantillon d'un sujet ; et

b) la détermination d'un niveau relatif d'expression desdits gènes par rapport à un niveau de référence, où l'expression modifiée desdits gènes par rapport à un niveau de référence indique que le sujet est à risque d'avoir le cancer de la prostate ou le cancer de la prostate agressif.

FIG. 1

**FIG. 2**

**FIG. 3**

FIG. 4

**FIG. 5**

**FIG. 6**

**FIG. 7**

**FIG. 8**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61785375 B
- EP 2373816 A
- WO 2012115885 A
- WO 2010065940 A
- WO 2006056766 A
- WO 2005113816 A
- WO 2012092336 A
- EP 2373816 A2
- WO 2012115885 A1
- WO 2010065940 A1
- WO 2006056766 A2
- WO 2005113816 A2
- WO 2012092336 A2
- WO 61785375 A

**Non-patent literature cited in the description**

- **CARTER et al.** *JCO,* 10 December 2012
- **AUSUBEL et al.** Current protocols in molecular biolog. John Wiley & Sons Ltd, 2003
- **CARTER et al.** *J. Clin. Oncol.,* 2012, vol. 30, 4294-4296
- **EPSTEIN et al.** Update on the Gleason grading system for prostate cancer: results of an international consensus conference of urologic pathologists. *Adv. Anat. Pathol.,* 2006, vol. 13 (I), 57-9
- **SAMBROOK et al.** Molecular cloning: A laboratory manual. Cold Spring Harbor Laboratory Press, 1989
- Use of a combination of biomarkers in serum and urine to improve detection of prostate cancer. **PRIOR et al.** World Journal of Urology. Springer, 2010, vol. 28, 681-686
- Beyond PSA: The Next Generation of Prostate Cancer Biomarkers. **PRENSNER et al.** Science Translational Medicine. American Association of the Advancement of Science, 2012, vol. 4, 54-64
- **GUYON et al.** A Four-Gene Expression Signature for Prostate Cancer Cells Consisting of UAP1, PDLIM5, IMPDH2, and HSPD1. *Urotoday International Journal,* 2009, vol. 02 (04), 1944-5784